# EUROPEAN PATENT SPECIFICATION

(11) **EP 2 793 855 B1**
(45) Date of publication and mention of the grant of the patent: **16.12.2015**
(21) Application number: 12826687.1
(22) Date of filing: 20.12.2012
(51) Int. Cl.: A61K 9/00, A61K 31/00, A61K 47/00

(54) **FLURBIPROFEN FORMULATIONS**
FLURBIPROFEN-FORMULIERUNGEN
FORMULATIONS DE FLURBIPROFÈNE

(30) Priority: 23.12.2011 TR 201112836
(43) Date of publication of application: 29.10.2014
(73) Proprietor: Sanovel Ilac Sanayi Ve Ticaret Anonim Sirketi, 34460 Istanbul (TR)
(72) Inventor: CIFTER, Umit, 34460 Istanbul (TR); TURKYILMAZ, Ali, 34460 Istanbul (TR); PEHLIVAN AKALIN, Nur, 34460 Istanbul (TR); BALCI, Ilayda, 34460 Istanbul (TR)
(74) Representative: Sevinç, Erkan
(86) International application number: PCT/TR2012/000245
(87) International publication number: WO 2013/095320

(56) References cited:
- GB-A- 2 432 526
- KR-A- 20070 059 758
- KR-A- 20110 136 256
- US-A- 4 996 209
- US-A1- 2004 039 366
- KENJIROU HIGASHI: "Simultaneous dissolution of naproxen and flurbiprofen from a novel ternary gamma-cyclodextrin complex", CHEMICAL AND PHARMACEUTICAL BULLETIN, PHARMACEUTICAL SOCIETY OF JAPAN, JP, vol. 58, no. 5, 1 May 2010 (2010-05-01), pages 769-772, XP009168502, ISSN: 0009-2363
- BASF: "Soluplus -Technical information", INTERNET CITATION, July 2010 (2010-07), pages 1-8, XP002666808, Retrieved from the Internet: URL:http://www.pharma-ingredients.basf.com /Statements/Technical%20Inf ormations/EN/Pharma%20Solutions/03_090801e _Soluplus.pdf [retrieved on 2012-01-10]

## Description

### Field of Invention

The present invention relates to novel pharmaceutical formulations of flurbiprofen or a pharmaceutically acceptable salt thereof, having anti-inflammatory, analgesic, and antipyretic activities.

The present invention particularly relates to orally-administered pharmaceutical formulations of flurbiprofen, having anti-inflammatory, analgesic, and antipyretic activities. The solubility, dissolution rate, and stability of said formulation are improved based on the excipients comprised therein.

### Background of Invention

Flurbiprofen is a propionic acid derivative, also known as NSAID (non-steroidal anti-inflammatory drug), having analgesic and anti-inflammatory activities. Its chemical structure is illustrated with Formula 1 given below.

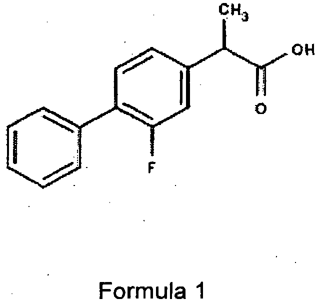

Flurbiprofen is used for alleviating pain in muscle-skeleton system and joint disorders such as ankylosing spondylitis, osteoarthritis, and rheumatoid arthritis, in soft tissue injuries such as sprains and strains, in postoperative cases, and in painful and severe menstruation and migraine. Flurbiprofen is further used as a lozenge in the symptomatic amelioration of sore throats.

Flurbiprofen sodium is used in eye drops for preventing intraoperative miosis, as well as for controlling the inflammation of the eye's anterior layer following surgery. Flurbiprofen axetil is administered via intravenous injection against severe pains in some countries.

The application WO 98/52545 relates to pharmaceutical compositions comprising a formulation of flurbiprofen with a therapeutically effective amount of one or more active ingredients selected from an antihistamine, a cough suppressant, a decongestant, an expectorant, a muscle relaxant, a centrally acting analgesic, a local anesthetic, an antibacterial compound, an antiviral compound, an antibiotic compound, an antifungal compound, minerals and vitamins and/or a burn-masking amount of an agent which has a warming effect on the mucosa of the throat.

The patent US05807568 discloses a topically-administered formulation comprising flurbiprofen as the active agent.

The patent WO9523596 discloses a flurbiprofen solution in a C2-4 alcohol.

The use of flurbiprofen in treating local pains and inflammations may cause a problem especially for those who have gastrointestinal system disorders. Preventing the systemic side effects of flurbiprofen is quite important in terms of patient compliance. Various coating processes have been performed to prevent such side effects. The coatings, however, may cause problems in terms of solubility and thus bioavailability. Enhancing the absorption rate both provides ease of application and increases the molecule's efficiency.

Another problem in relation to flurbiprofen is that this molecule is weakly soluble in water. This, in turn, directly effects the bioavailability, and therefore the solubility and dissolution rate have to be increased.

Another problem in relation to the active agent is stability, which emerges under the influence of ambient and physical conditions, as is the case with many other active agents. The active agent is influenced both from temperature, air, and humidity conditions, and from the solvents used while they are formulated. When they are exposed to air and humidity, said active agents degrade structurally and develop chemical behavioral changes. The stability of the products developed is not at a desired level and the shelf life thereof is shortened. In addition, these active agents are reactive against the excipients employed in developing the formulations containing the same. This, in turn, causes impurities to occur in the formulations and leads to the inclusion of undesired components into the formulations. It is of critical importance in terms of the formulation to use those excipients and methods which do not lead to said problems.

In result, a novelty is required in the art of pharmaceutical formulations having anti-inflammatory, analgesic, and antipyretic activities due to the aforesaid drawbacks.

### Object and Brief Description of Invention

The present invention provides an easily-administrable flurbiprofen formulation, eliminating all problems referred to above and bringing additional advantages to the relevant prior art.

Accordingly, the main object of the present invention is to obtain at least one stable formulation with anti-inflammatory, analgesic, and antipyretic activities.

Another object of the present invention is to provide a formulation having a desired solubility and dissolution rate, and therefore a desired level of bioavailability, with this formulation comprising flurbiprofen produced by means of a hot-melt method.

A further object of the present invention is to eliminate the need for any liquid solvent, including water.

Another object of the present invention is to obtain a uniform formulation content.

A further object of the present invention is to develop a formulation not leading to flowability-related problems during production.

A pharmaceutical formulation is developed to carry out all objects, referred to above and to emerge from the following detailed description.

According to a preferred embodiment of the present invention, said novelty is realized with flurbiprofen and polyvinylcaprolactam-polyvinyl acetate-polyethylene glycol graft copolymer.

According to another preferred embodiment of the present invention, said formulation is obtained by means of a hot-melt method not giving place to any liquid solvent during the granulation phase.

According to a preferred embodiment of the present invention, the proportion of flurbiprofen to polyvinylcaprolactam-polyvinyl acetate-polyethylene glycol graft copolymer is in the range of 0.1 to 10, preferably 0.15 to 5, and more preferably 0.2 to 2.

Another preferred embodiment of the present invention comprises at least one or more excipient(s).

According to a preferred embodiment of the present invention, said excipient(s) comprise(s) at least one or a properly-proportioned mixture of diluents, binders, disintegrants, glidants, lubricants, and plasticizers.

According to a preferred embodiment of the present invention, the mean particle size (d₅₀) of the granules obtained by means of the hot-melt method is in the range of 100 - 1500 µm, preferably 150 - 1000 µm, and more preferably 250 - 800 µm.

According to a preferred embodiment, the present invention also comprises at least one or a mixture of polyvinyl alcohol-polyethylene glycol copolymer, polyoxyethylene-polyoxypropylene block copolymer, stearyl macrogol glyceride, polyethylene glycol, povidone, cationic methacrylate, copovidone, methacrylic acid copolymer derivatives, cellulose acetate phthalate, acetylated monoglyceride, dibutyl tartrate, diethyl phthalate, dimethyl phthalate, glycerin, propylene glycol, stearic acid, triacetine, triacetine citrate and tripropionin. Polymers with a low glass transition temperature are preferred in the formulation according to the present invention.

In a preferred embodiment of the present invention, said disintegrant is at least one or a mixture of polyvinylpyrrolidone and sodium starch glycolate.

In a preferred embodiment of the present invention, said glidant is colloidal silicon dioxide.

In a preferred embodiment of the present invention, said lubricant preferably comprises at least one or a mixture of polyethylene glycol and magnesium stearate.

In a preferred embodiment of the present invention, said plasticizer comprises preferably at least one or a mixture of castor oil, glycerin, citrate esters (acetyl tri-n-butyl citrate, acetyl triethyl citrate, tri-n-butyl citrate, triethyl citrate) dibutyl sebacate, triacetine, diethyl phthalate, low molecular weight polyethylene glycols. The use of a plasticizer serves to reduce the glass transition temperature of the polymer and to increase the stability of the active agent used in the formulation.

In a preferred embodiment according to the present invention, said pharmaceutical formulation consisting of
a. 15 to 70% by weight of flurbiprofen or a pharmaceutically acceptable salt thereof,
b. 5 to 80% by weight of polyvinylcaprolactam-polyvinyl acetate-polyethylene glycol graft copolymer,
c. 0.5 to 25% by weight of croscarmellose sodium,
d. 0.1 to 10% by weight of colloidal silicon dioxide,
e. 0.1 to 10% by weight of magnesium stearate,
f. 0.1 to 10% by weight of plasticizer.

Another preferred embodiment according to the present invention provides a method for preparing said pharmaceutical formulation, this method comprising the steps of
a. mixing flurbiprofen, polyvinylcaprolactam-polyvinyl acetate-polyethylene glycol graft copolymer and plasticizer together, melting this mixture, and passing it through an extruder or sieve,
b. adding first croscarmellose sodium and colloidal silicon dioxide, and then magnesium stearate to the granules obtained and mixing the same,
c. performing a compression step on this powder mixture obtained in a tablet machine, or filling this powder mixture into capsules.

In another preferred embodiment of the present invention, said formulation is in the form of a tablet, capsule, or sachet.

### Detailed Description of Invention

### Example 1: Capsule or tablet

| **Ingredients** | **% amount (mg)** |
|---|---|
| Flurbiprofen | 15 - 70% |
| polyvinylcaprolactam-polyvinyl acetate-polyethylene glycol graft copolymer | 5 - 80% |
| croscarmellose sodium | 0.5 - 25% |
| colloidal silicon dioxide | 0.1 - 10% |
| magnesium stearate | 0.1 - 10% |
| plasticizer | 0.1 - 10% |

This formulation is produced as follows. Flurbiprofen, plasticizer and polyvinylcaprolactam-polyvinyl acetate-polyethylene glycol graft copolymer are mixed together, this mixture is melted and passed through an extruder or sieve. Into the granules obtained above, first croscarmellose sodium and colloidal silicon dioxide, and then magnesium stearate are added and the resulting mixture is mixed. A compression step is performed on this powder mixture in a tablet machine, or this powder mixture is filled into capsules. The tablets are coated preferably with a humidity-barrier coating material, such as Opadry amb/Kollicoat IR.

With this invention, a stable formulation is surprisingly obtained which has a high solubility and a high dissolution rate. Said formulation comprises flurbiprofen and polyvinylcaprolactam-polyvinyl acetate-polyethylene glycol greft copolymer or additionally at least one or a properly-proportioned mixture of polyvinyl alcohol-polyethylene glycol copolymer, polyoxyethylene-polyoxypropylene block copolymer, stearyl macrogol glyceride, polyethylene glycol, povidone, cationic methacrylate, copovidone, methacrylic acid copolymer derivatives, cellulose acetate phthalate, acetylated monoglyceride, dibutyl tartrate, diethyl phthalate, dimethyl phthalate, glycerin, propylene glycol, stearic acid, triacetine, triacetine citrate and tripropionin. The method described above both serves to provide a uniform formulation content, and eliminates the need for any liquid solvent including water. Any flowability-related problems encountered during production are prevented as well. In said formulation, the proportion of flurbiprofen to polyvinylcaprolactam-polyvinyl acetate-polyethylene glycol graft copolymer is in the range of 0.1 to 10, preferably 0.15 to 5, and more preferably 0.2 to 2. These ranges allow to achieve the desired dissolution rate and solubility. Polymers with low glass transition temperature and melting point are used in said formulation. On the other hand, using a plasticizer which reduces the glass transition temperature increases the stability of the active agent. The plasticizer used in a hot-melt method drops down the glass transition temperature of the polymers used in hot-melting, and thus allows to formulate the active agent at lower temperatures. In result, the formulation is made more stable.

The pharmaceutical compositions according to the present invention may also comprise one or more pharmaceutically acceptable excipient(s). Such pharmaceutically acceptable excipients include, but are not limited to fillers, glidants, lubricants, disintegrants, surface active agents etc. and the mixtures thereof.

The present invention is for use in mammalians and particularly in humans for the prevention or treatment of pain, arthralgia, toothache, myalgia, miosis inhibition, ankylosing spondylitis, osteoarthritis, rheumatoid arthritis and other muscle-skeleton system and joint disorders, soft tissue injuries such as sprains and strains, postoperative pains, painful and severe menstruation, migraine, and sore throat.

In this context, the term formulation may both correspond to a formulation, and to a combined meaning of the formulation and the package or blister in which the formulation is stored.

In this context, the term particle comprises a powder, granule, or a pellet.

It is also possible to use the following additional excipients in this formulation.

Diluents, e.g. at least one or a mixture of lactose, microcrystalline cellulose, starch, mannitol, calcium phosphate anhydrate, calcium phosphate dihydrate, calcium phosphate trihydrate, dibasic calcium phosphate, calcium carbonate, calcium sulfate, carboxymethyl cellulose calcium, powdered cellulose, cellulose acetate, pregelatinized starch, lactose monohydrate, corn starch.

Binders, e.g. at least one or a mixture of polymethacrylate, glyceryl behenate, polyvinylpyrrolidone (povidone), hydroxypropyl methyl cellulose (HPMC), hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose and other cellulose derivatives, polyethylene oxide, gelatin, starch, xanthan gum, guar gum, alginate, carrageen, pectin, carbomer, cellulose acetate phthalate, hydroxypropyl starch, hydroxyethyl methyl cellulose, polaxomer, polyethylene glycol (PEG).

Lubricants, e.g. at least one or a mixture of sodium stearyl fumarate, polyethylene glycol, stearic acid, metal stearates, boric acid, sodium chloride benzoate and acetate, sodium or magnesium lauryl sulfate, etc..

Preservatives, e.g. at least one or a mixture of methylparaben and propylparaben and salts thereof (e.g. sodium or potassium salts), sodium benzoate, citric acid, benzoic acid, butylated hydroxytoluene and butylated hydroxyanisole, etc..

Surface active agents, e.g. at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate, etc..

## Claims

1. A pharmaceutical formulation, **characterized by** comprising flurbiprofen and polyvinylcaprolactam-polyvinyl acetate-polyethylene glycol graft copolymer.

2. The pharmaceutical formulation according to Claim 1, wherein said formulation is obtained by means of a hot-melt method not involving any liquid solvent during the granulation phase.

3. The pharmaceutical formulation according to any of the preceding claims, wherein the proportion of flurbiprofen to polyvinylcaprolactam-polyvinyl acetate-polyethylene glycol graft copolymer is in the range of 0.1 to 10, preferably 0.15 to 5, and more preferably 0.2 to 2.

4. The pharmaceutical formulation according to any of the preceding claims, further comprising at least one or more than one excipient.

5. The pharmaceutical formulation according to any of the preceding claims, wherein said excipient comprises at least one or a mixture of diluents, binders, disintegrants, glidants, lubricants, and plasticizers.

6. The pharmaceutical formulation according to any of the preceding claims, wherein the mean particle size (d₅₀) of the granules obtained by means of the hot-melt method is in the range of 100 - 1500 µm, preferably 150 - 1000 µm, and more preferably 250 - 800 µm.

7. The pharmaceutical formulation according to any of the preceding claims, further comprising at least one or a mixture of polyvinyl alcohol-polyethylene glycol copolymer, polyoxyethylene-polyoxypropylene block copolymer, stearyl macrogol glyceride, polyethylene glycol, povidone, cationic methacrylate, copovidone, methacrylic acid copolymer derivatives, cellulose acetate phthalate, acetylated monoglyceride, dibutyl tartrate, diethyl phthalate, dimethyl phthalate, glycerin, propylene glycol, stearic acid, triacetine, triacetine citrate and tripropionin.

8. The pharmaceutical formulation according to any of the preceding claims, wherein said disintegrant is at least one or a mixture of croscarmellose sodium and sodium starch glycolate.

9. The pharmaceutical formulation according to any of the preceding claims, wherein said glidant is colloidal silicon dioxide.

10. The pharmaceutical formulation according to any of the preceding claims, wherein said lubricants preferably comprise at least one or a mixture of polyethylene glycol and magnesium stearate.

11. The pharmaceutical formulation according to any of the preceding claims, wherein said plasticizer comprises preferably at least one or a mixture of castor oil, glycerin, citrate esters (acetyl tri-n-butyl citrate, acetyl triethyl citrate, tri-n-butyl citrate, triethyl citrate) dibutyl sebacate, triacetine, diethyl phthalate, low molecular weight polyethylene glycols.

12. The pharmaceutical formulation according to any of the preceding claims, consisting of
a. 15 to 70% by weight of flurbiprofen or a pharmaceutically acceptable salt thereof,
b. 5 to 80% by weight of polyvinylcaprolactam-polyvinyl acetate-polyethylene glycol graft copolymer,
c. 0.5 to 25% by weight of croscarmellose sodium,
d. 0.1 to 10% by weight of colloidal silicon dioxide,
e. 0.1 to 10% by weight of magnesium stearate,
f. 0.1 to 10% by weight of plasticizer.

13. A method for preparing a pharmaceutical formulation according to any of the preceding claims, comprising the steps of
a. mixing flurbiprofen, plasticizer and polyvinylcaprolactam-polyvinyl acetate-polyethylene glycol graft copolymer together, melting this mixture, and passing it through an extruder or sieve,
b. adding first croscarmellose sodium and colloidal silicon dioxide, and then magnesium stearate to the granules obtained and mixing the same,
c. performing a compression step on this powder mixture in a tablet machine, or filling this powder mixture into capsules.

## Patentansprüche

1. Pharmazeutische Zubereitung, **dadurch gekennzeichnet, dass** sie Flurbiprofen und Polyvinylcaprolactam-Polyvinylacetat Polyethylenglycol Pfropfcopolymer aufweist.

2. Pharmazeutische Zubereitung nach Anspruch 1, wobei die Zubereitung durch ein Heißschmelzverfahren erhalten wird, an welchem kein Lösemittel während der Granulationsphase beteiligt ist.

3. Pharmazeutische Zubereitung nach einem der vorangehenden Ansprüche, wobei das Verhältnis von Flurbiprofen zu Polyvinylcaprolactam-Polyvinylacetat Polyethylenglycol Pfropfcopolymer im Bereich von 0,1 zu 10, vorzugsweise 0,15 zu 5, und besonders bevorzugt 0,2 zu 2 liegt.

4. Pharmazeutische Zubereitung nach einem der voranstehenden Ansprüche, welche mindestens einen oder mehr als einen Hilfsstoff aufweist.

5. Pharmazeutische Zubereitung nach einem der voranstehenden Ansprüche, wobei der Hilfsstoff mindestens ein oder eine Mischung von Verdünnungsmitteln, Bindemitteln, Zersetzungshilfen, Gleitstoffen, Schmierstoffen und Weichmachern aufweist.

6. Pharmazeutische Zubereitung nach einem der voranstehenden Ansprüche, wobei die Durchschnittspartikelgröße (d₅₀) der Granulate, welche mittels des Heißschmelzverfahrens erhalten wurden, im Bereich von 100 bis 1500 µm, vorzugsweise 150 bis 1000 µm, und besonders bevorzugt 250 bis 800 µm ist.

7. Pharmazeutische Zubereitung nach einem der voranstehenden Ansprüche, welche mindestens ein oder eine Mischung von Polyvinylalkohol-Polyethylenglycolcopolymer, Polyoxyethylen-Polyoxypropylen Blockcopolymer, Stearyl Makrogol glycerid, Polyethylenglycol, Povidon, kationisches Methacrylat, Copovidon, Methacrylsäurecopolimer Derivate, Zelluloseacetatphtalat, acetyliertes Monoglycerid, Dibutyltartrat, Diethylphtalat, Dimethylphtalat, Glycerin, Propylenglycol, Stearinsäure, Triacetin, Triacetincitrat und Tripropioin aufweist.

8. Pharmazeutische Zubereitung nach einem der voranstehenden Ansprüche, wobei die Zersetzungshilfe mindestens ein oder eine Mischung von Croscarmellose-Natrium und Natriumglycolatstärke ist.

9. Pharmazeutische Zubereitung nach einem der voranstehenden Ansprüche, wobei der Gleitstoff kolloidales Siliziumdioxid ist.

10. Pharmazeutische Zubereitung nach einem der voranstehenden Ansprüche, wobei das Schmiermittel vorzugsweise mindestens ein oder eine Mischung von Polyethylenglycol und Magnesiumstearat aufweist.

11. Pharmazeutische Zubereitung nach einem der voranstehenden Ansprüche, wobei der Weichmacher vorzugsweise mindestens ein oder ein Mischung von Rizinusöl, Glycerin, Citratesthern (Acetyl-tri-n-buthylcitrat, Acetyltriethylcitrat, Tri-n-buthylzitrat, Triethylcitrat) Dibutylsebacat, Triazetin, Diethylphtalat, niedermolekulare Polyethylenglycole aufweist.

12. Pharmazeutische Zubereitung nach einem der voranstehenden Ansprüche, bestehend aus
a) 15 bis 70 Gew.% Flurbiprofen oder einem pharmazeutisch geeigneten Salz davon,
b) 5 bis 80 Gew.% Polyvinylcaprolactam-Polyvinylacetat Polyethylenglycol Pfropfcopolymer,
c) 0,5 bis 25 Gew.% vernetztem Croscarmellose-Natrium,
d) 0,1 bis 10 Gew.% kolloidalem Silikondioxid,
e) 0,1 bis 10 Gew.% Magnesiumstearat
f) 0,1 bis 10 Gew.% Weichmacher.

13. Verfahren zum Herstellen einer pharmazeutischen Zubereitung nach einem der voranstehenden Ansprüche, mit den Schritten:
a) Mischen von Flurbiprofen, Weichmacher und Polyvinylcaprolactam-Polyvinylacetat Polyethylenglycol Pfropfcopolimer, Schmelzen dieser Mischung und Durchleiten derselben durch einen Extruder oder Sieb,
b) zuerst Zugabe von vernetztem Croscarmellose-Natrium und kolloidalem Siliziumdioxid und anschließend Magnesiumstearat zu den Granulaten und Durchmischen derselben,
c) Durchführen eines Kompressionsschritts an der Pudermischung in einer Tablettierungsmaschine oder Füllen des Puders in Kapseln.

## Revendications

1. Formulation pharmaceutique, **caractérisée par le fait qu'**elle comprend du flurbiprofène et un copolymère greffé de polyvinylcaprolactame-polyacétate de vinyle-polyéthylène glycol.

2. Formulation pharmaceutique selon la revendication 1, dans laquelle ladite formulation est obtenue au moyen d'un procédé de thermo-fusion n'impliquant pas de solvant liquide pendant la phase de granulation.

3. Formulation pharmaceutique selon l'une quelconque des revendications précédentes, dans laquelle la proportion de flurbiprofène par rapport au copolymère greffé de polyvinylcaprolactame-polyacétate de vinyle-polyéthylène glycol se situe dans la plage de 0,1 à 10, de préférence de 0,15 à 5, et plus préférablement de 0,2 à 2.

4. Formulation pharmaceutique selon l'une quelconque des revendications précédentes, comprenant également au moins un excipient ou plusieurs excipients.

5. Formulation pharmaceutique selon l'une quelconque des revendications précédentes, dans laquelle ledit excipient comprend au moins un composant parmi les diluants, les liants, les agents de désintégration, les agents de glissement, les lubrifiants et les plastifiants ou un mélange de ceux-ci.

6. Formulation pharmaceutique selon l'une quelconque des revendications précédentes, dans laquelle la taille moyenne de particules (d₅₀) des granulés obtenus au moyen du procédé de thermo-fusion se situe dans la plage de 100 - 1 500 µm, de préférence de 150 - 1 000 µm et plus préférablement de 250 - 800 µm.

7. Formulation pharmaceutique selon l'une quelconque des revendications précédentes, comprenant également un des composants suivants ou un mélange des composants suivants : copolymère d'alcool polyvinylique-polyéthylène glycol, copolymère séquencé de polyoxyéthylène-polyoxypropylène, glycéride de macrogol stéarylique, polyéthylène glycol, povidone, méthacrylate cationique, copovidone, dérivés de copolymère d'acide méthacrylique, acétophtalate de cellulose, monoglycéride acétylé, tartrate de dibutyle, phtalate de diéthyle, phtalate de diméthyle, glycérine, propylène glycol, acide stéarique, triacétine, citrate de triacétine et tripropionine.

8. Formulation pharmaceutique selon l'une quelconque des revendications précédentes, dans laquelle ledit agent de désintégration est au moins un composant suivant ou un mélange des composants suivants : croscarmellose sodique et glycolate d'amidon sodique.

9. Formulation pharmaceutique selon l'une quelconque des revendications précédentes, dans laquelle ledit agent de glissement est du dioxyde de silicium colloïdal.

10. Formulation pharmaceutique selon l'une quelconque des revendications précédentes, dans laquelle lesdits lubrifiants comprennent de préférence au moins un des éléments suivants ou un mélange des éléments suivants : polyéthylène glycol et stéarate de magnésium.

11. Formulation pharmaceutique selon l'une quelconque des revendications précédentes, dans laquelle ledit plastifiant comprend de préférence au moins un des éléments suivants ou un mélange des éléments suivants : huile de ricin, glycérine, esters de citrate (citrate d'acétyle tri-n-butyle, citrate d'acétyle triéthyle, citrate de tri-n-butyle, citrate de triéthyle) sébacate de dibutyle, triacétine, phtalate de diéthyle, polyéthylène glycols à faible poids moléculaire.

12. Formulation pharmaceutique selon l'une quelconque des revendications précédentes, composée de
a. 15 à 70 % en poids de flurbiprofène ou d'un de ses sels pharmaceutiquement acceptables,
b. 5 à 80 % en poids de copolymère greffé de polyvinylcaprolactame-polyacétate de vinyle-polyéthylène glycol,
c. 0,5 à 25 % en poids de croscarmellose sodique,
d. 0,1 à 10 % en poids de dioxyde de silicium colloïdal,
e. 0,1 à 10 % en poids de stéarate de magnésium,
f. 0,1 à 10 % en poids de plastifiant.

13. Procédé de préparation d'une formulation pharmaceutique selon l'une quelconque des revendications précédentes, comprenant les étapes suivantes :
a. mixage de flurbiprofène, de plastifiant et de copolymère greffé de polyvinylcaprolactame-polyacétate de vinyle-polyéthylène glycol, fusion de ce mélange et passage du mélange à travers une extrudeuse ou un tamis,
b. ajout d'abord de croscarmellose sodique et de dioxyde de silicium colloïdal, et puis de stéarate de magnésium aux granulés obtenus et mixage de l'ensemble,
c. réalisation d'une étape de compression sur ce mélange de poudres dans une comprimeuse ou remplissage de capsules avec ce mélange de poudres.
